# EUROPEAN PATENT APPLICATION

(11) **EP 1 403 037 A1**
(43) Date of publication of application: **31.03.2004**
(21) Application number: 02724684.2
(22) Date of filing: 02.05.2002
(51) Int. Cl.: B32B 5/24, A61F 13/514

(54) **HIGHLY PERMEABLE AND WATER RESISTANT BARRIER SHEET, AND ABSORBER PRODUCT USING THE BARRIER SHEET**

(30) Priority: 02.05.2001 JP 2001135237
(71) Applicant: Japan Absorbent Technology Institute, Chuo-ku, Tokyo 103-0007 (JP)
(72) Inventor: SUZUKI, Migaku, Chuo-ku, Tokyo 103-0007 (JP)
(74) Representative: Gervasi, Gemma, Dr.
(86) International application number: PCT/JP2002/004401
(87) International publication number: WO 2002/090106

(57) **Abstract**

A highly permeable and water resistant barrier sheet, comprising a porous buffer sheet and a water resistant nonwoven cloth of 100 mmH₂O or higher in water resistance connected integrally with each other and combined with a permeable sheet-like absorber to form an absorbing core so as to make an absorber product highly permeable, wherein the porous buffer sheet is a perforated film formed desirably in an irregular structure having funnel-shaped perforated structures with top and bottom parts different in diameter from each other and, when the diameter thereof on the shorter diameter side is 1 mm or shorter when a hole is approximated in a complete round shape and the number of holes is 20/cm² or more.

## Description

### Field of art

The present invention relates to a highly air permeable and water resistant barrier sheet comprising a conjugate of a porous buffer sheet and a water resistant nonwoven fabric.

### Background technology

To an absorbent product, skin rash and irritation poses a big problem as accompanied by the wearing of the absorbent product. Various artifices have been presented to overcome such problem such as providing contrivances to the surface materials of the absorbent product, i.e. providing a air permeable zone in the side portion or the end portion of the product or using as the back sheet various materials in combination. No ultimate solutions have been made so far.

So as to give the so-called breathable back sheet which is a back sheet imparted permeability, many proposals have already been made such as methods of utilizing a laminated member of a microfibrillated web or a gel blocking behavior. The only commercially available effective means is to use a so-called air permeable PE sheet as the back sheet. The permeability of normally available air permeable film is as low as 3.0 to 5.0 kg/24 hr·m² as expressed in terms of WVTR (Water Vapor Transfer Ratio) ASTM E 96-63 F, E 96-80B. This air permeability nearly equals to 500∼100 sec/100 ml as expressed in term of the Gurley Method.

In the meantime, according to certain studies on the skin discomfort due to the stuffiness of underwears worn, gap of 2 mm or more is required between the surface of an underwear and the surface of the skin together with a high permeability to make surface air stream existent by virtue of the bellows effect for preventing such discomfort. A normally worn underwear has such permeability that 100 ml of air is made to pass in less than 1.0 second even if it is of a dense fabric as expressed by the Gurley method (ISO 5636/5). If expressed in terms of the permeability by ASTM D-737 for comparing with air permeable films, the permeability of a normal underwear fabric is usually 1 m³ or more per min · m², and further if it is a thin fabric, its permeability is 10 m³ or more per min · m² and even when it is in a wet condition, the permeability is still at least 0.5 m³ or more per min · m². The permeability of this level is nearly 100∼300 times as high as that of a air permeable film discussed above. Hence, it is understandable how it is difficult to have an absorbent product sufficiently air permeable not to cause the skin discomfort due to the stuffiness of an underwear or skin rash.

Besides, since a typical absorbent product represented by a disposable diaper is today designed to fit the body as less gap in between as possible, it has no such gap in between for having the bellows effect work with little air stream on the surface. In addition, since the absorbent body itself of the absorbent product has a thick layer of pulp/SAP, what permeability the air permeable film has does not work as expected after it has absorbed bodily fluid. This is the case today. It is quite natural that such absorbent product likely to result in such condition is worn, the humidity and the temperature inside the product is increased so that the skin discomfort due to the stuffiness of the product or skin rash are likely to be caused.

In addition, a proposal has been made to provide ventilation holes on the sides or the front and the back or the end of an absorbent product taking risks of causing possible leakage, and a new problem is caused that the effluvium of body fluids comes out of such holes.

That is to say, in order to make possible an absorbent product which is highly air permeable and in addition less likely to cause skin discomfort due to the stuffiness of an absorbent product and skin rash, although it is important to have more air permeable back sheet, it is necessary but not sufficient, and it is also necessary to impart a stably air permeable structure to the absorbent body itself of the absorbent product.

### Disclosure of the invention

An object of the present invention is to provide novel highly air permeable and water resistant barrier sheets wherein a high level of permeability and at the same time a high level of prevention of leakage which are conflicting with each other are made to be compatible.

According to the present invention, a highly air permeable and water resistant barrier sheet characterized in that the barrier sheet is comprised by a porous buffer sheet the diameter of whose pores is 1 mm or less and the number of whose pores is 10 or more per cm² and a water resistant nonwoven fabric whose water resistance is 100 mmH₂O or higher as bonded in an integrated form.

A preferable porous buffer sheet is a highly air permeable foamed sheet having continuous air bubbles whose number of cells is 20 or more per 25 mm and whose thickness is 3 mm or less.

Another preferable porous buffer sheet is an apertured film with many funnel-shaped hollow juts formed having different diameters in the top portion and the bottom portion, and, when the pore of the top portion of the hollow jut approximates a perfect round shape, the diameter is 1 mm or less and the number of pores is 10 or more per cm².

Also, a preferable water resistant nonwoven fabric is a multi-layered member of spun-bonded web and melt-blown web formed as a material base of PE, PP, PET or polyurethane or any of their derivatives either alone or mixed in any arbitrary combination. The configuration of the layers of the multi-layered member of spun-bonded web (S) and melt-blown web (M) is SMS, SMMS, SMMM or SMSMS.

Furthermore, the present invention provides an absorbent product having as an absorbent core including as an absorbent component at least one of SAP and fluff pulp in powder, particulate or fibrous form, wherein an highly air permeable and water resistant barrier sheet is provided as disposed in contact with the layer of said absorbent component, said highly air permeable and water resistant barrier sheet comprising a composite of a porous buffer sheet and a water resistant nonwoven fabric whose water resistance is 100 mmH₂O or higher, said porous buffer sheet being an apertured film with many funnel-shaped hollow juts formed with the diameter of its top portion being different from that of the bottom portion, the diameter of the top portion of the hollow juts being 1 mm or less when the diameter approximates a perfect round shape and the number of pores being 10 or more per cm² and the top portion of the hollow juts being disposed in contact with said absorbent core.

In an absorbent product like this, said buffer sheet is disposed as covering the whole undersurface, a part of the sides and a part of the face of said absorbent core, with the undersurface of said absorbent core bonded as integrated with said water resistant nonwoven fabric.

Said buffer sheet may cover the whole undersurface and the sides of said absorbent core, bonded in its undersurface as integrated with said water resistant nonwoven fabric. Alternatively, said buffer sheet may be disposed as developed on the undersurface of said absorbent core, bonded in its entire surface as integrated with said water resistant nonwoven fabric so that the buffer sheet may function as a back sheet.

The portion of the whole absorbing surface of said absorbent core where discharged bodily waste is fed in excess of the water retaining capability or pressure is concentrated locally may be reinforced by means of a non-air permeable film whose water resistance is high or a air permeable film whose water resistance is high.

In said absorbent product, said water resistant film and said buffer sheet may be bonded as integrated with said water resistant nonwoven fabric as combined with each other only in their respective edges.

In said absorbent product, the thickness of said absorbent water resistant sheet is 0.5 mm or less, said water resistant nonwoven fabric as its component is SMS or SMMS of 30 g/m² to 10 g/m², said hydrophilic sheet is tissue mainly comprising wood pulp of 30 g/m² to 10 g/m² and said SAP includes particle SAP of 80 g/m² to 210 g/m² whose particle size is 500 µm or less.

50% or more of the whole weight of said absorbent core is preferably constituted by SAP. More preferably, said absorbent core is a sheet-like conjugate comprising a nonwoven fabric and SAP as carried by said nonwoven fabric.

Another preferable absorbent core is a sheet-like absorbent body formed by air laid method into a sheet shape as comprises a mixture of fluff pulp and SAP.

It should be noted that in a highly air permeable and water resistant barrier sheet according to the present invention the "water resistance" needs to be 100 mmH₂O or higher, or preferably 150 mmH₂O or higher, if expressed in terms of the water resistance as generally used in this field of technology and that the water resistance of normally used film such as 2000 mmH₂O or higher is not required and such level of water resistance as can be said to be water permeability is sufficient.

### Brief Description of Drawings

Fig. 1 is an oblique perspective drawing showing an example of an apertured film as applied to a highly air permeable and water resistant barrier sheet according to the present invention, the film being convexo-concave.
Fig. 2 is an illustrative drawing showing the behavior of fluids movement in case the convexo-concave film according to Fig. 1 is combined with an absorbent core.
Fig. 3 is an oblique perspective drawing showing the appearance of the convexo-concave apertured film having an opening provided on the top portion as applied to a highly air permeable and water resistant barrier sheet according to the present invention.
Fig. 4 is a partially cutout drawing of the convexo-concave apertured film of Fi. 3.
Fig. 5 is illustrative drawings showing different shapes and distributions of openings formed on the top of the evaginated portion of the convexo-concave film.
Figs. 6 (a), (b) and (c) are oblique perspective drawings showing respectively different positions of openings formed on each evaginated portion of the convexo-concave film.
Fig. 7 is an illustrative drawing exemplifying change of the thickness of polyurethane foam as is compressed thermally or absorbs water.
Fig. 8 is a drawing showing the configuration wherein an apertured film and a water resistant nonwoven fabric are combined with an absorbent core.
Fig. 9 is a pattern diagram showing two kinds of gaps (A, B) existent in the structures of a water resistant nonwoven fabric and a buffer sheet as combined.
Fig. 10 is a flow diagram showing the cycle of an absorbent core absorbing fluids from its start of absorption to completion in the structures of a buffer sheet according to Fig. 9 and a water resistant nonwoven fabric with the absorbent core.
Fig. 11(a) is a plan view showing a sheet-like absorbent body comprising three components of an absorbent layer mainly comprising a highly absorbent resin, a nonwoven fabric substrate carrying the highly absorbent resin and a binding material component binding the highly absorbent resins with each other and the highly absorbent resin with the nonwoven fabric substrate. Fig. 11 (b) is a cross-sectional view of a sheet-like absorbent body according to Fig. 11 (a).
Fig. 12 (a) is a cross-sectional view showing a product model wherein a buffer sheet covers the whole undersurface and both side portions of an absorbent core and further a part of the face portion of the absorbent core and is made a highly air permeable and water resistant barrier sheet as bound in its undersurface portion in an integrate form with a water resistant nonwoven fabric.
Fig. 12 (b) is a cross-sectional view showing a product model wherein a buffer sheet covers the whole undersurface and both side portions of an absorbent core and is made a highly air permeable and water resistant barrier sheet as bound in its undersurface portion in an integrate form with a water resistant nonwoven fabric.
Fig. 12 (c) is a cross-sectional view showing a product model wherein a highly air permeable and water resistant barrier sheet constituted by a buffer sheet and a water resistant nonwoven fabric as bound in an integrated form with each other is disposed in the undersurface of an absorbent core.
Fig. 13 (a) is an illustrative view showing a structure wherein on both sides of an absorbent core a sheet-like absorbent body is quadruplicated in a bank shape. Fig. 13 (b) is an illustrative view showing an example wherein a liquid imair permeable reinforcing film is disposed on the side portions.
Fig. 14 (a) is an illustrative view showing an absorbent product of a structure wherein in the center portion of an absorbent core a sheet-like absorbent body is tripled. Fig. 14 (b) is an illustrated view showing an absorbent product of a structure wherein a reinforcing film is disposed in the center portion of an absorbent core.
Fig. 15 is a schematic longitudinal sectional view showing an example of an absorbent product wherein, with a buffer sheet omitted for the portion where a reinforcing film exists, a buffer sheet and a film portion are partially overlapped with each other to be bound with a water resistant nonwoven fabric.
Fig. 16 is a schematic longitudinal sectional view showing a back-up sheet as applied to an absorbent product according to the present invention.
Fig. 17 is a schematic longitudinal sectional view showing an example of an absorbent product according to the present invention with a back-up sheet added to the outside of a highly air permeable and water resistant barrier sheet.
Fig. 18 is a schematic longitudinal sectional view showing another example of an absorbent product according to the present invention with a back-up sheet added to this inside of a highly air permeable and water resistant barrier sheet.
Fig. 19 is a side elevation view showing the whole of an apparatus for evaluating water resistance as applied in evaluating water resistance.
Fig. 20 is a side elevation view showing the part of the apparatus of Fig. 19 as enlarged where an evaluation sample is mounted.
Fig. 21 is a cross-section view showing an absorbent product model constituted by liquid distribution unit (LDU) being combined with a highly air permeable and water resistant barrier sheet according to the present invention, which is in turn folded with an absorbent core.
Fig. 22 is an illustrative diagram showing the material balance obtained by analyzing the existential condition of fluids absorbed in an absorbing test of a product model of an absorbent product with no buffer sheet used.
Fig. 23 is an illustrative diagram showing the material balance obtained by analyzing the existential condition of fluids absorbed in an absorbing test of a product model of an absorbent product with a buffer sheet used.

### Best Mode of Practicing the Present Invention

An highly air permeable and water resistant barrier sheet according to the present invention based on a novel concept obtained by binding a porous buffer sheet with an a water resistant nonwoven fabric will be explained first, and then an absorbent product obtained by combining said barrier sheet and an absorbent core will be explained.

### Porous Buffer Sheet

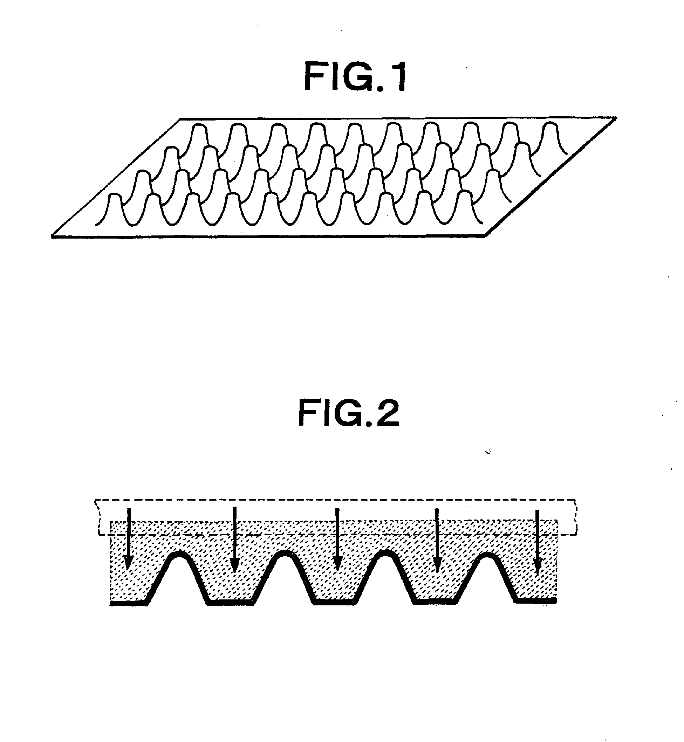

A first constituent component of a highly air permeable and water resistant barrier sheet according to the present invention is a porous buffer sheet. This buffer sheet is used in an absorbent product with one surface thereof being disposed directly under an absorbent core and the other surface being bound with a water resistant nonwoven fabric in an integrated form, so that the following conditions need to be satisfied:
(1) The buffer sheet needs to be sufficiently air permeable not to affect the permeability of the absorbent core above and of the water resistant nonwoven fabric below.
(2) The buffer sheet needs to have the function of trapping temporarily water yet to be absorbed (so-called free water) passing through the absorbent core without being absorbed by SAP.
   This trapping function is referred to as a bottom acquisition function (temporary retention in the bottom) so that it is distinguished from a conventional surface acquisition function (temporary retention on the surface) wherein the buffer sheet is disposed on the upper portion of the absorbent core in proximity with the top sheet. Water yet to be absorbed trapped by a buffer sheet having this bottom acquisition function is reabsorbed by the SAP layer of the absorbent core above as time passes, and then made stabilized.
   Here, the bottom acquisition function possessed by the buffer sheet is as mentioned above a function of temporarily retaining fluids from the absorbent core yet to be absorbed in the bottom of the absorbent core. SAP which is a main component of the absorbent core leaks temporarily out of the absorbent core since it is slow in the initial absorbing speed, and fluids coming out below as get in contact with a conventional film-like back sheet flow on the surface of the back sheet and build up in the lowest portion by means of the law of gravity so the fluids come out as leak through any gap. Hence, such fluids need to be trapped so that they are made uniformly distributed and not moved.
   For example, in case a sheet so formed as to give convexes and concaves as shown in Fig. 1 is disposed as a back sheet in contact with an absorbent core, fluids are divided depending on the depths and the number of concaves as shown in Fig. 2 so that and the convexo-concave sheet gets to have a function of trapping fluids as a result and the fluids are pooled uniformly on the whole surface of the sheet. Such function is generally called as the bottom acquisition function.
   It is noted that a convexo-concave sheet as shown in Figs. 1 and 2 has a good bottom acquisition function, but is not air permeable so such sheet is not suitable for the object of the present invention.
(3) The buffer sheet needs to have some degree of water resistance.
   It is preferable that the amount to be transferred to the water resistant nonwoven fabric below of water yet to be absorbed as trapped on the buffer sheet or in the gaps of the buffer sheet is made as less as possible. The buffer sheet having such function of stopping water needs to have water resistance of such level as not to hamper the high permeability.
(4) The buffer sheet needs to have a structure of many cells or convexes and concaves for defining and finely segmenting fluids.
   It is required that free water (water yet to be absorbed) coming out of the absorbent core and temporarily retained by the buffer sheet is not pooled locally, but retained on the whole portion of the buffer sheet in contact with the fluids so that the fluids are defined and finely segmented in streams by means of a dividing structure possessed by the buffer sheet resulting in no local load imposed on the water resistant nonwoven fabric.
(5) The buffer sheet needs to have a so-called buffer effect, in other words a cushion effect, that load and pressure imposed to the absorbent core are alleviated so water pressure as applied to the water resistant nonwoven fabric below is reduced.

### Configuration of Porous Buffer Sheet

It is preferable that, for a porous buffer sheet to be highly air permeable and to exert a function as the stopper of fluids by means of its water resistance, it is porous and at the same time its pores are very fine. At the same time, the buffer sheet needs to be hydrophobic, or preferably constituted by a hydrophobic material, so that the surface tension is made to operate. The pore size is preferably 1 mm or less, and more preferably 0.5 mm or less. Examples of materials constituting the porous body may be mono-polymers, copolymers and their blends such as PE, PP, PET, synthetic rubber, urethane and EVA or their sheet formed products surface treated with such water repellent agent as silicone and Teflon (registered trademark).

For the function of trapping free water (water yet to be absorbed), so-called bottom acquisition function, to be fully fulfilled, the buffer sheet needs to have a structure that it is bulky with its inside area being large and has the function of physically retaining fluids sufficiently. For such bottom acquisition function to be great enough, it is preferable if said in the simplest way that the buffer sheet is as bulky as possible with its apparent specific gravity being small. With such buffer sheet used, however, an absorbent product becomes thicker. It is an indispensable requirement that an absorbent product needs to be compact. Such thick absorbent products do not meet the marketing requirements. Accordingly, there should be inherent limitations on the thickness of the products. Thicker and bulkier buffer sheet fulfills the object of the present invention in some way because such buffer sheet has a cushion effect of preventing weight imposed on an absorbent core from being added to a water resistant nonwoven fabric, but, when considering such extremely thin absorbent product the thickness whose absorbent core is 5 mm or less, the thickness of a buffer sheet to be used for such extremely thin product is at least 3 mm or less and preferably 1 mm or less. A buffer sheet, in order that it has 1 mm or less thickness and its buffer effect as mentioned above is stably exerted, needs to be stiff sufficiently that it does not deform significantly when pressure is applied to it or is required to keep a property that when it is dry before use it is kept thin, 1 mm or less, as compressed and deformed, but its bulkiness is recovered when it absorbs moisture or water.

Another important function of a buffer sheet is to make uniform the flow of the distribution and diversion of fluids. The porous structure of a buffer sheet as used in the present invention is preferably such that, unlike a uniform bulky nonwoven fabric as obtained by a through air process used very often as an acquisition layer in a usual absorbent product, it has a lot of convexo-concave portions like a plastic film formed as having convexes and concaves or a lot of cells like a foam having continuous air bubbles.

Typical examples having such structure are a foamed urethane sheet having continuous air bubbles and an apertured plastic film having a convexo-concave structure.

A representative example of such apertured film having a convexo-concave structure is an apertured film with many convexes and concaves each of a so-called funnel shape having different pore sizes of the top and the bottom portion. This apertured film is generally used as a highly stain-free surface member with its side of larger pore size facing upwards disposed on the side of receiving fluids so that the flowing-in of fluids is made easier and at the same time the back-flow from downwards of fluids is prevented. Also, a composite material as made by conjugating a bulky liquid air permeable nonwoven fabric on its surface is described in Japanese patent application 2000-501883 (of Tredegar) and used as a surface material.

In the present invention an important feature is that an apertured film is disposed in a reverse direction, i.e. with its side of smaller pore size facing upwards and as supporting an absorbent core from downwards in the bottom of the absorbent core so that some resistance is given against the flow-in of fluids and at the same time the moving of fluids from downwards is made likely to be easy. In other words, this type of configuration is required so that, even if fluids yet to be absorbed leaking from an absorbent core overflow out of a buffer sheet and as a result further leak also to the side of a water resistant nonwoven fabric from the pore, in case the leaking fluids are reabsorbed as the absorbing capacity of SAP is recovered, the fluids are made to flow easily to the absorbent core through the pore.

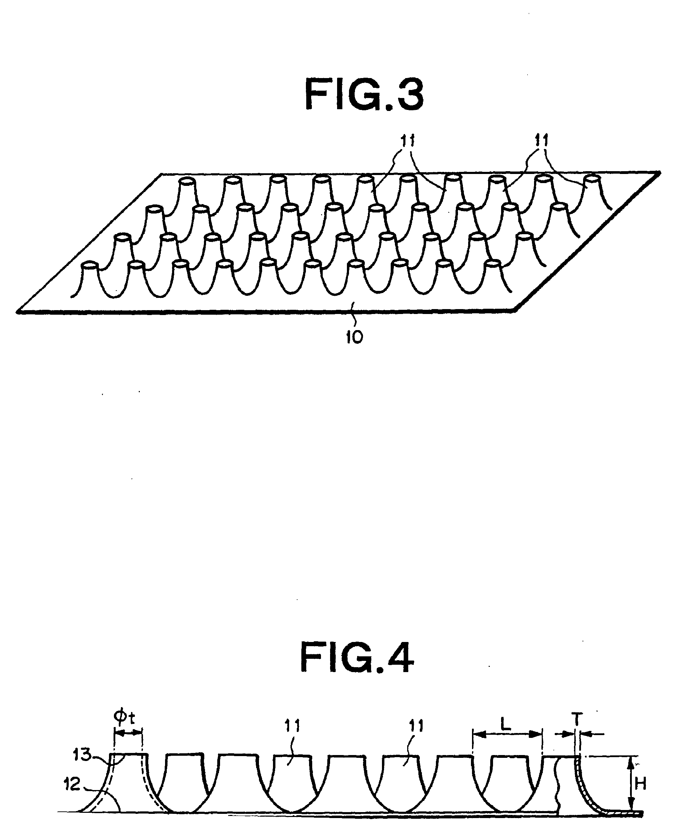

Fig. 3 shows the appearance of an apertured film 10 having convexes and concaves as used preferably in the present invention, and Fig. 4 is a cross-sectional view of a part thereof as enlarged. The apertured film 10 as shown has many funnel-shaped evaginated portions 11 as formed in an appropriate distribution density, and on the side of larger diameter (bottom portion) of the evaginated portion 11 a bottom pore 12 having a diameter φb is formed and on the side of smaller diameter (top portion) of the evaginated portion 11 a top pore 13 having a smaller diameter φt is formed, respectively. The ratio of diameter φb to φt (φb / φt) is preferably selected from the range of 1.1 to 3.0. Also, the distance between the adjacent evaginated portions 11 is indicated by L, the height by H and the thickness by T. The preferable height is 3 mm or less. The thickness T is the thickness of a material film and preferably 0.01 to 0.5 mm. It is noted that the thickness and the height were measured using Daiei Kagaku Seiki's FS 60 A under the minimum loading condition of 3 g/cm². It was intended to indicate the thickness under a condition as near to no-load as possible.

What is important with the apertured film is, first of all, the size of the smaller pore formed on the top portion of the funnel-shaped evaginated portion 11. This pore size is preferably as small as possible in the range that no air permeability is injured because, when fluids absorbed by an absorbent core are beyond the capability of the absorbent core, the pore becomes an excretory pore for excreting the fluids overflowing from the pore to the water resistant nonwoven fabric of the back sheet side or the back-up sheet side. A preferable pore size is 2 mm or less, and more preferably 1 mm or less if represented by a diameter when the shape of the pore is made to approximate a complete round shape. Under this condition, fluids (yet to be absorbed) weeping down to the bottom layer through the absorbent core are trapped by the concaves of the convexo-concave apertured film and are, until overflowing from there, prevented by the resistance coming from the surface tension of the pores directly flowing out to the water resistance nonwoven fabric. If the pore is beyond 2 mm, however, fluids transferred downwards from the absorbent core are made to bypass directly to the water resistant nonwoven fabric side through the pores of a buffer sheet without exhibiting its water resistance so that its trapping function, i.e. its bottom acquisition effect is made meaningless.

The number of the pores per a unit area of an apertured film is also an important factor with the present invention. An apertured film has less pores than a foamed sheet (to be described later), so the number of pores is important. Also, the number of pores has much to do with the number of diversions and distributions of fluids yet to be absorbed overf lowing to the water resistant nonwoven fabric. The number of pores if expressed in terms of the number of pores per cm² is preferably 10 /cm² or more and more preferably 20 to 300 /cm². If the number is less than 10 /cm², the distribution and diversion effect is hard to be uniform and thus the buffer effect becomes lower.

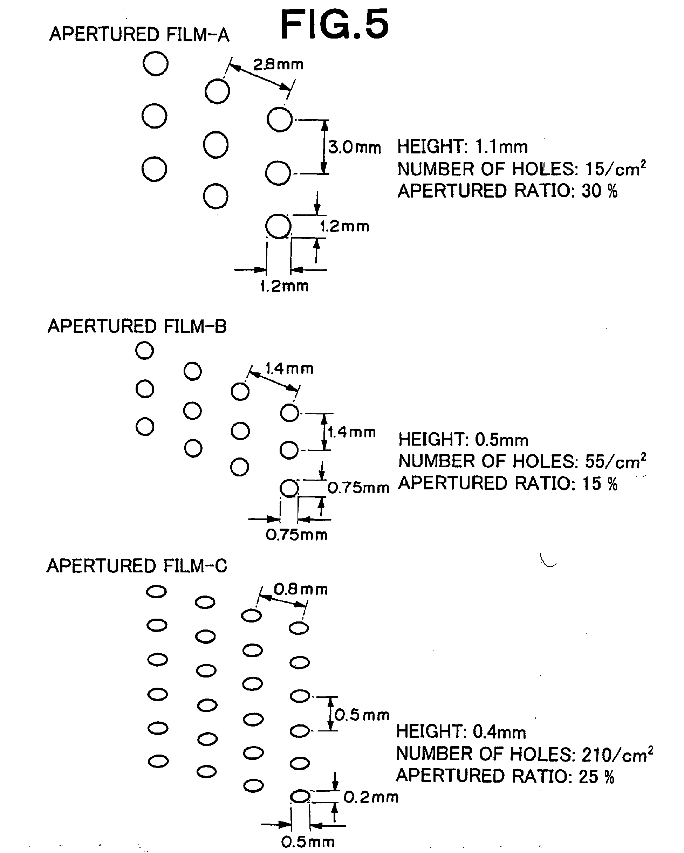

Fig. 5 shows examples of distribution of pores on the top portion of apertured films. All of apertured films A, B and C are of PE. Apertured film A, which is relatively coarsely apertured, is of Tredegar (USA), apertured film B, which is relatively finely apertured, is of Avgol (Israel) and apertured film C, which is more finely apertured, is of Tredegar.

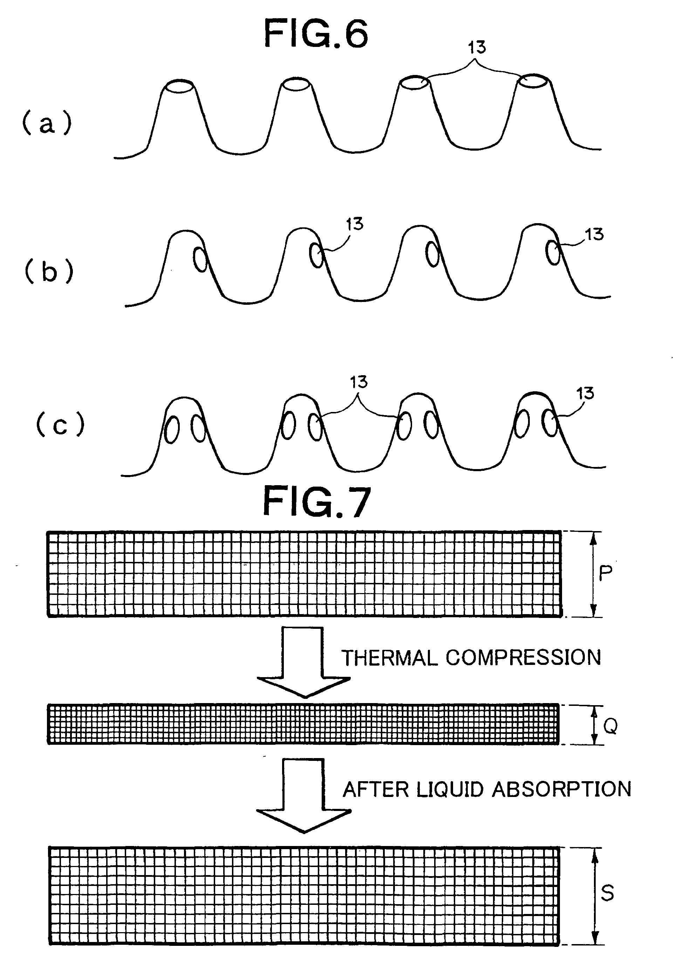

In the present invention, no particular restrictions are imposed on the shapes of pores formed on an apertured film, but the position of pores may somewhat influence the bottom acquisition function. In order to obtain better bottom acquisition effect, pores 13 are preferably positioned on the top of an evaginated portion as shown in Fig. 6 (a). In order to have higher water resistance under load, the pores are preferably disposed on the side of an evaginated portion as shown in Fig. 6 (b), in which case, as shown in Fig. 6 (c), two pores 13 may be formed on one evaginated portion and as required three or more pores may be formed on one evaginated portion. In case pores are formed on the side of an evaginated portion, the pores are made flattened in shape when deformed under load, which may sometimes be better.

On the other hand, a foamed sheet is a sheet of hydrophobic foam such as PE, PP, polyurethane, PET and synthetic rubber and should be air permeable having continuous air bubbles. In general, such foamed sheet has preferably an air permeability of at least 5 m³/min · m² (ASTM D-737) and, if expressed in the number of cells and the degree of foaming, the foamed sheet has preferably the following physical properties:
Porosity : 70% to 99%,
Number of cells : 20 cells / 25 mm to 80 cells / 25 mm, and
Degree of foaming : 15 to 50 times.

The thickness of a foamed sheet is, like an apertured film as above described, 3 mm or less and preferably 1 mm or less. A thicker than 3 mm sheet, however, may be preferably used if it is made thin as compressed under load.

In other words, a preferable bulky state of a foamed sheet means that it is kept as thin as possible not only when packaged as a absorbent product but also when opened or worn so that as accompanied by the absorbed swelling of an absorbent core as wetted when body fluids are discharged the sheet as a buffer sheet may be made swollen and bulky.

A buffer sheet may be such as is bulky having resilience under compression as described in Japanese Patent Application 2001-19777, but rather, preferably, is such as is likely to crumple, i.e. to deform to thin state permanently without its structure destroyed. Being made thin like this under compression is relatively easy in the case of materials such as foamed sponge. For example, a foam of a thermoplastic polymer such as PE and PP can be compressed down to a degree of one tenth to one fifth by being pressed at its thermal melting or lower temperature. Even a polyurethane foam whose heat plasticization temperature is high can be compressed down to a degree of one fifth by treating under high pressure as heated. Even a cellulose sponge of no thermoplasticity can be also compressed down to a degree of one fifth by treating under heat and compression as it is wetted. These compressed sponges recover nearly to their original level of bulkiness by means of the hydration of foamed cells if when used water is absorbed under the pressure of body weight.

Fig. 7 exemplifies change of the thickness of a polyurethane foam in the course of thermal compression and water absorption. In case the thickness P of a polyurethane foam yet to be compressed is for example 8 mm, the thickness is compressed down to 2 mm, i.e. a degree of one fourth of the original thickness, the thickness of the foam as compressed being called Q. The compressed foam stably keeps the 2 mm thickness when it is dry, but, after it is used as a buffer sheet and thus has absorbed water under compression, its thickness is recovered to a degree of 6 mm, the recovered thickness being called S. The thickness as buffer sheet in this case is indicated to be 2 mm as the thickness when it is dry.

### Water Resistant Nonwoven Fabric

Next, the concept and characteristics of a water resistant nonwoven fabric, a second component of a highly air permeable and water resistant barrier sheet, are explained.

A water resistant nonwoven fabric in combination with a buffer sheet as above described functions for trapping fluids coming out of the pores of the buffer sheet and preventing such fluids from leaking outside the system. Accordingly, materials constituting the water resistant nonwoven fabric are required to have considerably more finely porous than an aperturued film and a sponge. A nonwoven fabric of fine denier constituent fibers and of a dense construction meets such requirements.

Such nonwoven fabric is preferably a water resistant nonwoven fabric having a property to less wet to water being permeability resistant to some extent and as thin with uniform hydrophobicity and water repellency as possible. Examples of such nonwoven fabric are melt blown nonwoven fabric, spun bond nonwoven fabric and their conjugates SMS, SMM and SMSMS, of PE, PP, PET, polyurethane and their derivat ives or mixtures. The weight of such nonwoven fabric is preferably in the range of 10 g/m² to 30 g/m². The water resistance of such nonwoven fabric is preferably high, but, as described later, since it is important that it functions effectively in combination with a buffer sheet, the water resistance of a single layer of such nonwoven fabric is 100 mmH₂O or more and preferably 150 mmH₂O or more. In this sense, a preferable nonwoven fabric is SMS which is a composite of a spun bond (S) and a melt blown web of finer constituent fibers (M), and SMMS, SMMMS and SMSMS which are composites with a higher percentage of a melt blown layer and made uniform, and the denier of filaments constituting a spun bond layer is preferably 2 denir or less.

In order to stabilize the water resistance of a water resistant nonwoven fabric, the surface of the nonwoven fabric may be treated with a water repellent agent such as silicone and Teflon (registered trademark).

### Integration of Water Resistant Nonwoven Fabric with Buffer Sheet

In a highly air permeable and water resistant barrier sheet according to the present invention, it is necessary to join and integrate a water resistant nonwoven fabric as above described and a buffer sheet in a close contact with each other. For integrating them, a buffer sheet and a water resistant nonwoven fabric may be joined with an adhesive agent or a fusing agent, but it is preferably recommended that, in order not to inhibit the air permeability of a nonwoven fabric and a buffer sheet, they are joined with a bonding agent, a hot melt resin usually used for hygiene products being applied by means of a hot melt applicator such as a spray coater and a curtain coater to them as they are made in a fibrous state.

Alternatively, an apertured film / a melt blown composite or a foamed sheet / a melt blown composite which are manufactured by a coupled process of a manufacturing process of an apertured film or a foamed sheet and a melt blown process may be used.

A water resistant nonwoven fabric is used as it is in a composite laminated state of a buffer sheet as described above, and, by means of such laminated structure, becomes to possess such completely different properties from those of a single layer.

Table 1 below shows measurements of a single layered water resistant nonwoven fabric, a single layered buffer sheet and a barrier sheet comprising laminate structure of such single layers in terms of water resistance. From these results it is seen that, although the water resistance possessed by an apertured film as a buffer sheet is extremely low, the water resistance is made considerably high by lamination.

**Table 1**

| Constituent materials | | Water resistance (mmH₂O) |
|---|---|---|
| Water resistant nonwoven fabric | SMMS (22 g/m²) | 220 |
| Buffer sheet | Apertured film A | 5 |
| | Apertured film B | 12 |
| | Apertured film C | 25 |
| | Polyurethane foam | 30 |
| Barrier sheet | Apertured film A/SMMS | 260 |
| | Apertured film B/SMMS | 390 |
| | Apertured film C/SMMS | 430 |
| | Polyurethanefoam/SMMS | 330 |

The results shown in Table 1 show that the barrier sheet made by disposing SMMS as a water resistant nonwoven fabric to an apertured film B and to an apertured film C each of relatively fine pores exhibits very high water resistance.

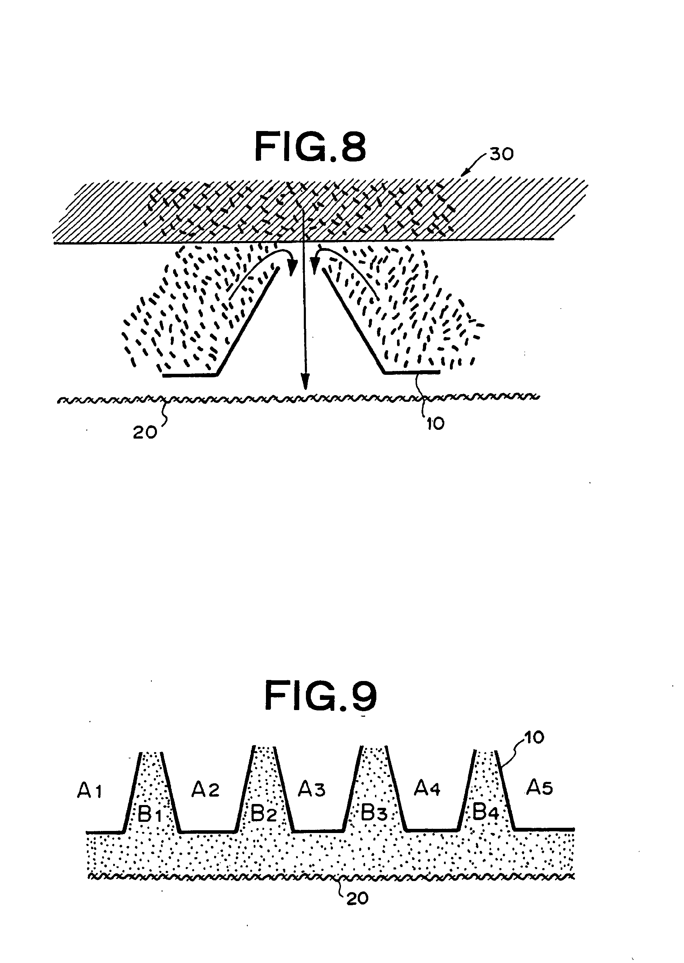

Next, an absorbent product constituted by combining a high air permeable and water resistant nonwoven barrier sheet with an absorbent core is explained with reference to Fig. 8. Fig. 8 shows a configuration relation among a water resistant nonwoven fabric 20, a apertured film 10 and an absorbent core 30 in combination. In this configuration, as shown in Table 1 for example, in case an apertured film 10 whose water resistance is 25 mmH₂O (an apertured film C of Fig. 5) is combined with a water resistant nonwoven fabric 20 comprising SMMS whose water resistance is 220 mmH₂O, the sum of the water resistances of the individual elements is only 245 mmH₂O, but the measurement of a joined and integrated composite is 430 mmH₂O, which is nearly two times as high as the former. One reason for this seems to be that, by employing a combined configuration of an apertured film and SMMS, a combined effect of the water resistant structure of the apertured film 10 and the layer of air existent is realized so that some or other water resistance alleviation effect is made to work on the water resistant nonwoven fabric 20.

### Mechanism of Trapped Fluids Reabsorbed

What is further important with a joined structure of a buffer sheet and a water resistant nonwoven fabric is a bottom acquisition effect and a mechanism of trapped fluids yet to be absorbed being reabsorbed by an absorbent core containing SAP.

Fig. 9 is a pattern diagram showing two kinds of air spaces existent in a structure of a water resistant nonwoven fabric 20 and a buffer sheet of an apertured film 10 as combined, i.e. an air space A existent in a concave portion of a buffer sheet, an apertured film, and an air space B existent between a water resistant nonwoven fabric and a convex portion of a buffer sheet of an apertured film 10.

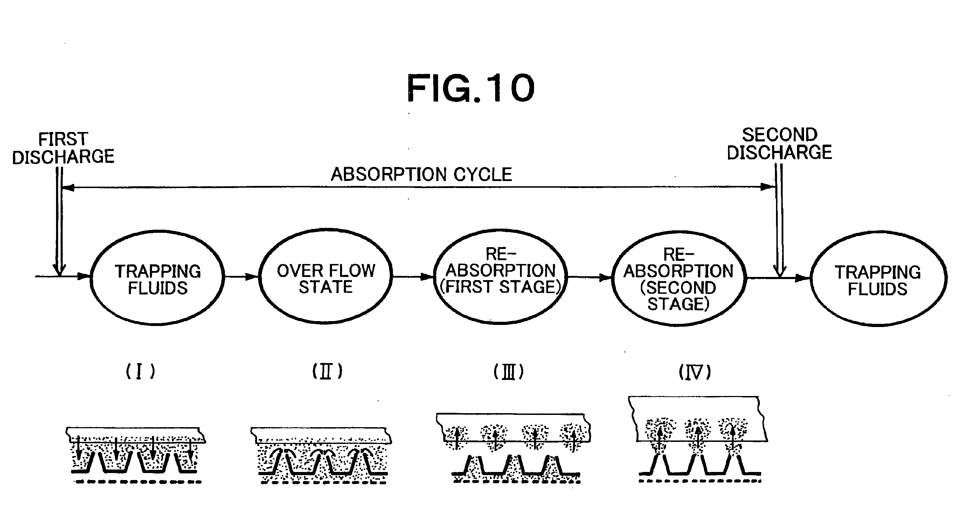

In addition, Fig. 10 shows an absorbing cycle example from the start to the end of absorption of fluids by an absorbent core in a configuration shown in Fig. 9. Body fluids discharged are first diffused onto the surface of an absorbent core and then absorbed partly by the SAP layer with fluids remaining not absorbed moving further to the bottom layer. Any fluids left yet to be absorbed resulting from the supply of body fluids in excess of the absorption by SAP are trapped first by portion A of the buffer sheet having many cell-shaped concaves so that it shows a state shown in Fig. 9 (I). If the amount of fluids yet to be absorbed is further more, portion A becomes filled and the fluids as diffusing all over the concaves and convexes overflow from the pores to fill portion B, which is a state as shown in Fig. 9 (II).

Since, as time passes, however, the SAP layer on the top provides a surplus capacity of absorption, fluids existent in portion A are first reabsorbed by the absorbent core. Then, the fluids in portion B are also reabsorbed after flowing back through the pores. This is as shown in Fig. 9 (III). Further, as time passes, almost all of the fluids in portions A and B are reabsorbed by the absorbent core, and both of portions A and B are recovered to their original spaces so that the portions are made ready to receive next coming discharged fluids. This is as shown in Fig. 9 (IV).

The load applied to the water resistant nonwoven fabric becomes highest when, as shown in Fig. 9 (II), both of portions A and B are filled and such load is alleviated as time passes. Even when both of the portions are filled as the load is at its maximum, the amount received as the water pressure from the trapped water is low like as 2 mm to 5 mmH₂O as the free water pressure so that the water resistant nonwoven fabric can function to prevent leakage.

The volume occupied percentages of portions A and B of a buffer sheet in bearing the expected functions are shown in Table 2 in the states of (I) to (IV) in Fig 9. State (I) means the state that 80 to 90 % of portion A alone out of 100 % of the respective capabilities of portions A and B is utilized, state (II) shows the state that 100 % of portions A and B is utilized when they are filled, state (III) means a state that while portion A starts to reabsorption and the half of the fluids has been reabsorbed in portion A, a part of portion B (20%) has been reabsorbed as affected by portion A. In state (IV), a state is shown in a model that almost all fluids have been reabsorbed and portions A and B are ready for the next discharge.

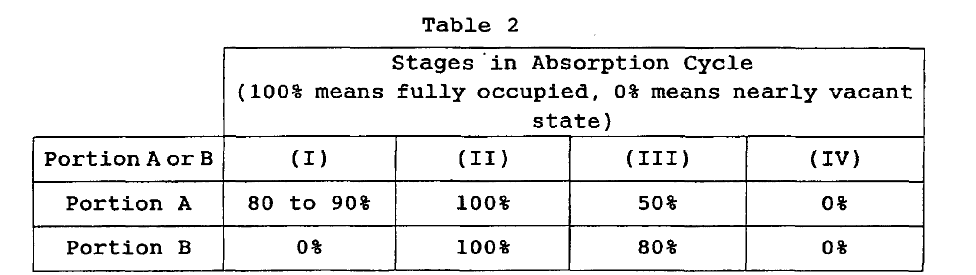

In the above schematic example the leakage prevention effects have been explained of a barrier sheet having a multi-layered of a buffer sheet and a water resistant nonwoven fabric in combination. Below an absorbent product as constituted by such barrier sheet in combination with an absorbent core is explained.

### Absorbent Core Preferable in Absorbent Product of Present Invention

One of preferable absorbent cores as used in absorbent products according to the present invention is an absorbent core containing a high percentage of SAP and that, for example, as recited in Japanese Patent Application Laid Open Hei 10-168230 and Japanese Patent Application Laid Open 2000-201975, such absorbent sheet as comprises three components of an absorbent layer mainly consisting of a highly absorbent resin, a nonwoven fabric substrate carrying the highly absorbent resin and a bonding agent for bonding the highly absorbent resins and the highly absorbent resin to the nonwoven fabric substrate.

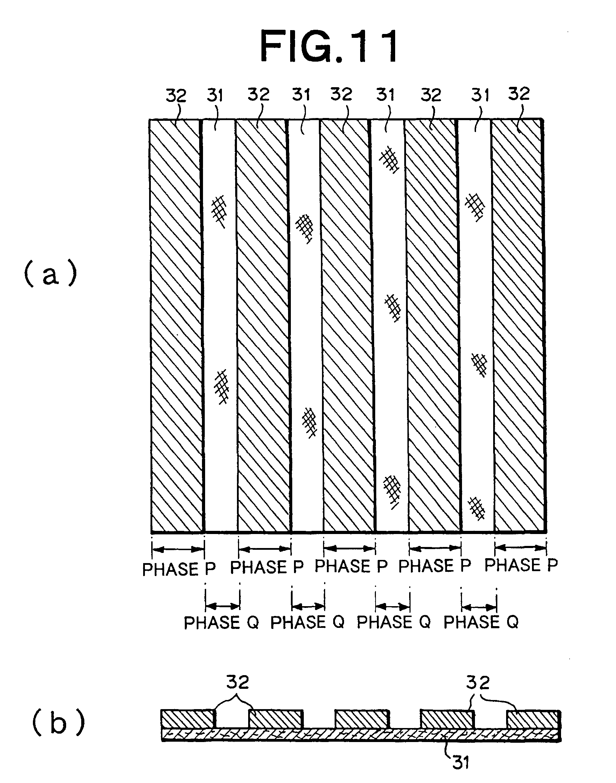

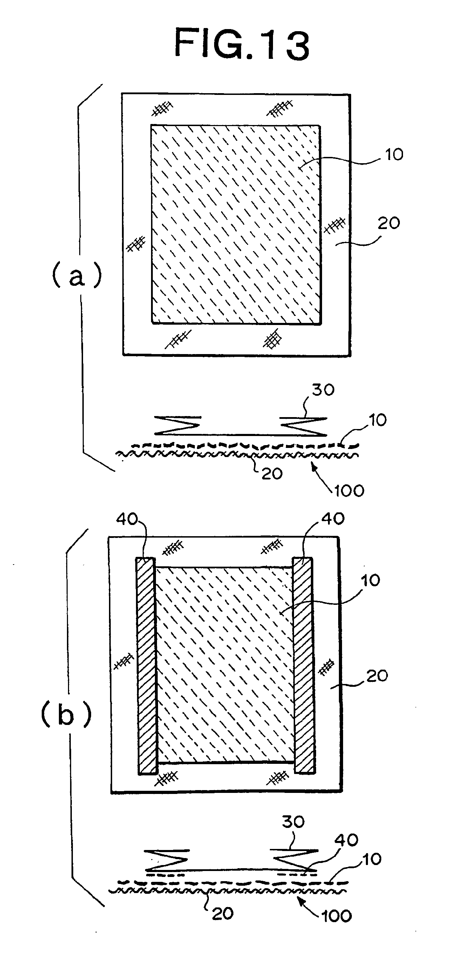

One example of such absorbent sheet is shown in Fig. 11. In Fig. 11 reference code 31 represents a nonwoven fabric substrate, and reference code 32 indicates SAP layer provided line zone state on the surface of this nonwoven fabric substrate. The zone where SAP layer exists functions as an absorbent zone phase (P phase) and the zone where no SAP layer exists functions as a diffusion/acquisition zone phase (Q phase) which is extremely low in air flow resistance. In this way an absorbent sheet wherein phases mutually different in functions are existent as continuous phases adjacent to each other is suitable to the present invention. The Q phase of this absorbent sheet has an air flow structure where air can pass freely before and after absorbing fluids.

Also, this absorbent sheet, since it has a SAP content of 80 to 90 %, falls within the concept of absorbent cores of so-called super super thin structure, and an absorbent core like this is very excellent in compactness but has a defect that its initial absorption speed is low. Hence, conventionally an method has been employed that on the top side of an absorbent core a surface acquisition layer of a bulky nonwoven fabric or fluff pulp is provided to trap fluids temporarily and then to supply fluids to the SAP layer. However if fluids are intended to be trapped only by the surface acquisition layer, the utilization of the SAP layer is reduced necessarily resulting in that the acquisition layer needs to be thicker and larger. Consequently, the product cost will be higher and the benefit of compactness will be forfeited. The present invention solves these problems also by providing a buffer sheet having a bottom acquisition function.

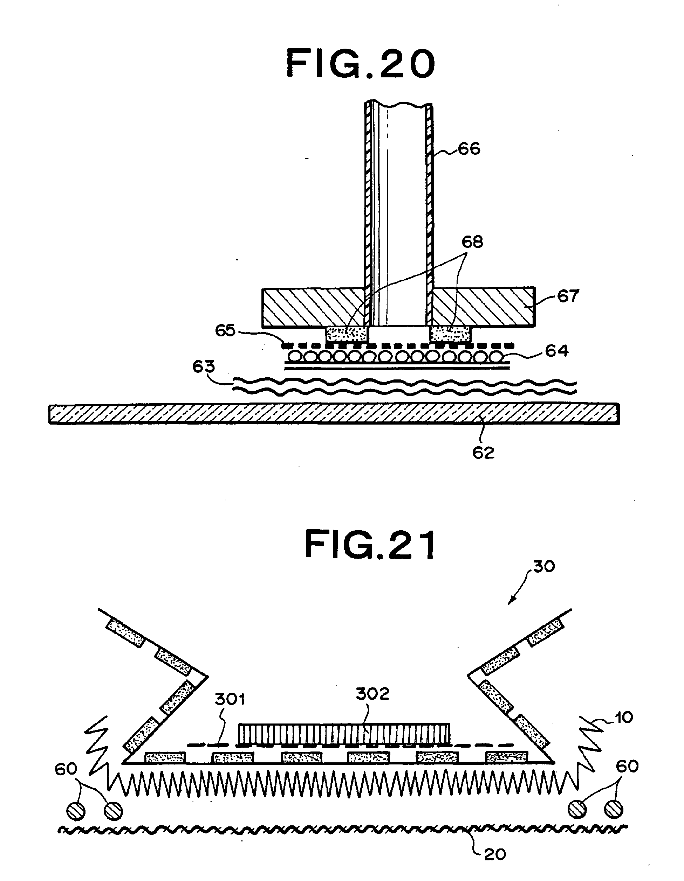

For example, the urinating rate of a healthy baby is approximately 100 ml/30 sec. Thus it is naturally required that any difference between the urinating rate and the absorbing rate of an absorbent core needs to be adjusted and, in such case, the present invention works effectively. One solution for adjusting such difference of the absorbing rate is to use Liquid distribution unit (LDU) and diffuse liquids rapidly all over the surface of an absorbent core so that the utilization of the area space may be increased as proposed by the inventors of the present invention in their Japanese Patent Application 2001-044494. Another solution to solve this problem is to eliminate the above-described absorbing rate difference by trapping fluids yet to be absorbed utilizing the bottom acquisition effect possessed by a buffer sheet. Thus, by combining the first solution of Liquid distribution unit (LDU) and the second solution of the buffer sheet, the absorption rate can be considerably improved. The specific effects of such means will be explained with reference to the embodiment of an absorbent product shown in Fig. 21.

### Configuration Relation of Absorbent Core to Barrier Sheet

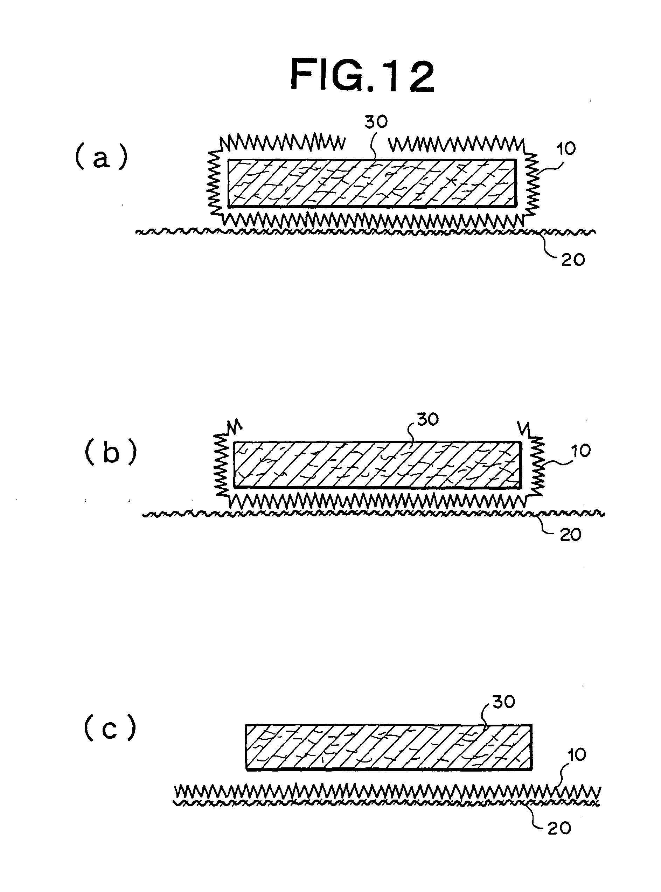

An embodiment of combining an absorbent sheet containing a high concentration of SAP as described above and a barrier sheet according to the present invention is explained with reference to Figs. 12.

In an example shown in Fig. 12 (a), an apertured PE film 10 used as a buffer sheet covers both sides of the under surface of an absorbent core 30 and a part of the surface portion of the absorbent core 30 whereby the apertured PE film 10 also functions so-called core wrapping covering a great part of the absorbent core. The under surface of the apertured film 10 is bonded to and integrated with SMMS of PP constituting a water resistant nonwoven fabric 20 and EVA type hot melt (not shown) to function as a highly air permeable and water resistant barrier sheet. An absorbent product having such absorbent core structure has a buffer sheet whose area of use is large, and the part existent on the top surface of the absorbent core functions as a Liquid distribution unit (LDU) and has multi functions operating as a core wrapping making easier the reabsorption of fluids in the regions from the side portions extending down to the under surface and further working in the under surface region as a back sheet as a highly air permeable and water resistant barrier sheet in a composite with a water resistant nonwoven fabric.

Fig. 12 (b) shows an example wherein an apertured PE film used as a buffer sheet covers the whole under surface and both sides of an absorbent core 30 and bonded and integrated in its under surface portion with SMMS of PP, a water resistant nonwoven fabric 20, by means of EVA type hot melt (not shown). In an absorbent product having such absorbent core structure, a buffer sheet, i.e. an apertured film 10, exhibits as a core wrapping function making easier the reabsorption of fluids in the regions from the side portions extending down to the under surface of the absorbent core 30 and fullfils in its under surface region the function of a back sheet as a highly air permeable and water resistant barrier sheet in a composite with a water resistant nonwoven fabric 20.

In an example shown in Fig. 12(c), a highly air permeable and water resistant barrier sheet wherein a buffer sheet comprising an apertured PE film and a water resistant nonwoven fabric comprising SMMS of PP are bonded to each other in an integrated form is disposed as developed widely on the under surface of an absorbent core 30 to function as a back-up sheet.

### Countermeasures when Load is Excessive to Highly Air permeable and Water Resistant Barrier Sheet

In a highly air permeable and water resistant barrier sheet having a structure according to the present invention, the retention of absorbed fluids of SAP, a main component of an absorbent core, is very important. There are such cases where body fluids may be discharged in excess of the retention of SAP, pressures coming from body fluids may be locally concentrated and leakage may take place in a region where SAP is overloaded. As against such cases it is effective to reinforce such regions beforehand with more water resistant non air permeable film or more water resistant highly air permeable film. The positions and the area of such reinforcing may be arbitrarily selected. It should be noted that if the positions are too many or the area is too large, the significance of existence of a barrier sheet according to the present invention is diminished.

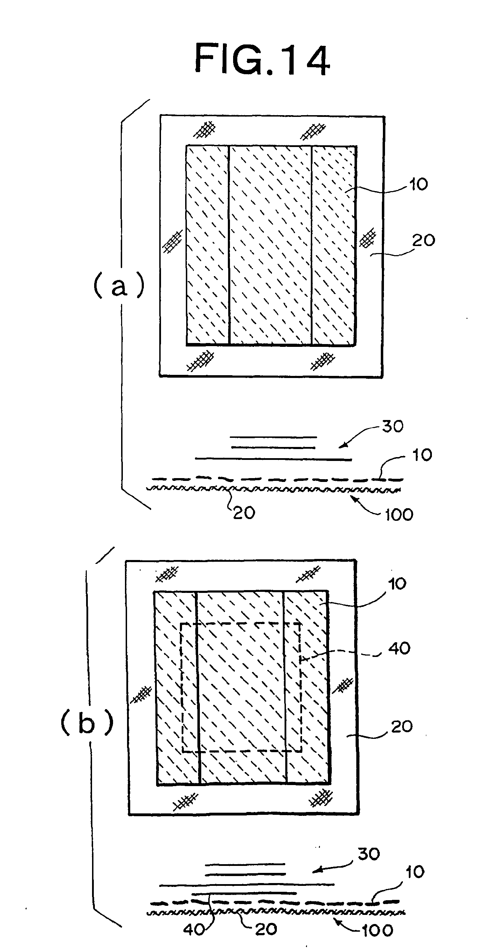

Figs. 13 and 14 show examples of reinforcing with a non-air permeable film or a highly water resistant air permeable film. In an example shown in Fig. 13 (a), the sides of an absorbent core are so structured that absorbent sheets are quadruplicated in the form of a bank. As baby and adult diapers are contemplated, the body weight is concentratedly applied on this quadruplicated portion so that there may be likely leakage on the sides. In such case, as shown in Fig. 13 (b), such leakage may be eliminated by disposing a liquid imair permeable reinforcing film 40 on each side. In the case of this example, a PE film of 15 µm thickness is disposed between a highly air permeable and water resistant barrier sheet 100 and an absorbent core 30. Of course, this film may be placed between an apertured film 10 constituting a highly air permeable and water resistant barrier sheet and a water resistant nonwoven fabric 20.

In an example shown in Fig. 14 (a), an absorbent core 30 is so structured that an absorbent sheet is trebled in the middle portion of the core. In this case, since the amount of fluids to be absorbed becomes larger and the load is concentrated in the middle portion, as shown in Fig. 14 (b), possible leakage may be eliminated by disposing a reinforcing film 40 in the middle portion. In the case of this example, the reinforcing film 40 is obtained by disposing a PE film of 15 µm thickness between a highly air permeable and water resistant barrier sheet and an absorbent core 30. The reinforcing may be either in a longitudinal direction or in a width direction.

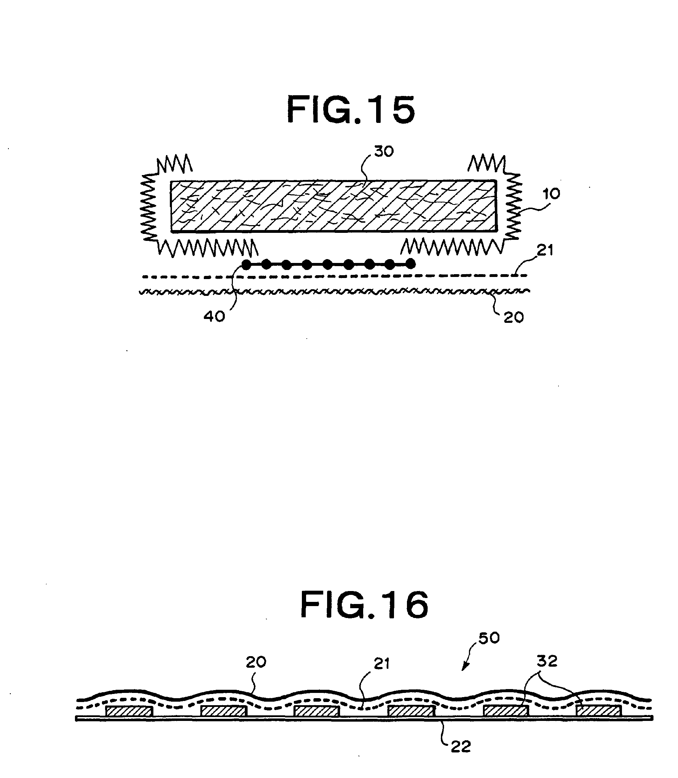

Some embodiments have thus been explained of adding film to a buffer sheet. It is an effective means that, since if a buffer sheet is folded on a film, the leakage prevention function is duplicated in that particular portion with a resulting increase in the cost, a buffer sheet in the portion where such film is existent is omitted and, as shown in Fig. 15, an apertured film 10 is folded partly on a reinforcing film 40, which are then bonded to a water resistant nonwoven fabric 20 with a hot melt 21, for use.

### Highly Air permeable and Water Resistant Barrier Sheet with Leakage Prevention Stability Improved

In case an adult diaper is used for incontinence applications or a baby diaper is used for nighttime applications where loading is high or in such systems where an absorbent body is pulp rich, since free fluids are always existent in abundance, it is necessary to maintain a stable leakage prevention effect. In this case, reinforcing with film as discussed above is not suitable since the reinforced portion is increased and the air permeability may be injured. In a case like this, the structure becomes complicated with a resulting increased cost, but one layer of an absorbent water resistant sheet is added as a back-up sheet so that, while the air permeability may be maintained, the leakage prevention may be secured. The absorbent water resistant sheet is an absorbent sheet having a coating pattern of SAP as shown in Fig. 11 made thinner and more flexible, which has a structure shown in Fig. 16. In other words, it has a structure where a hydrophilic sheet 22 of tissue having a water diffusing property is thinly coated with SAP 32 in line patterns to which coated surface a water resistant nonwoven fabric 20 is bonded by means of hot melt 21 resulting in a three-layered structure and thus, such structure has absorbency and at the same time has water resistance so that it is called an absorbent water resistant sheet 50. A back-up sheet is intended to work as a back up in an emergency leading to leakage, and thus preferably thin and flexible. The maximum thickness of the back-up sheet is 1 mm and preferably less than 0.5 mm.

An absorbent water resistant sheet 50 should be as thin and water resistant as possible. SAP used for the sheet is particulate with the particle size being 500 µm or less and preferably 300 µm or less. The amount of SAP to be used for coating is 100 g/m² or less and preferably 20 to 80 g/m² or less. Thus, a thin and flexible structure is preferable.

A water resistant nonwoven fabric may be, as explained above, SMS or SMMS comprising hydrophobic fibers, and in this example a relatively thin fabric of 13 g/m² is used.

A hydrophilic sheet is for diffusing leaking fluids uniformly, and a relatively thin sheet made of cellulose fibers such as wood pulp, rayon and cotton is used. The weight is preferably 30 to 10 g/m², and in this example tissue of 15 g/m² comprising wood pulp is used for constituting the hydrophilic sheet.

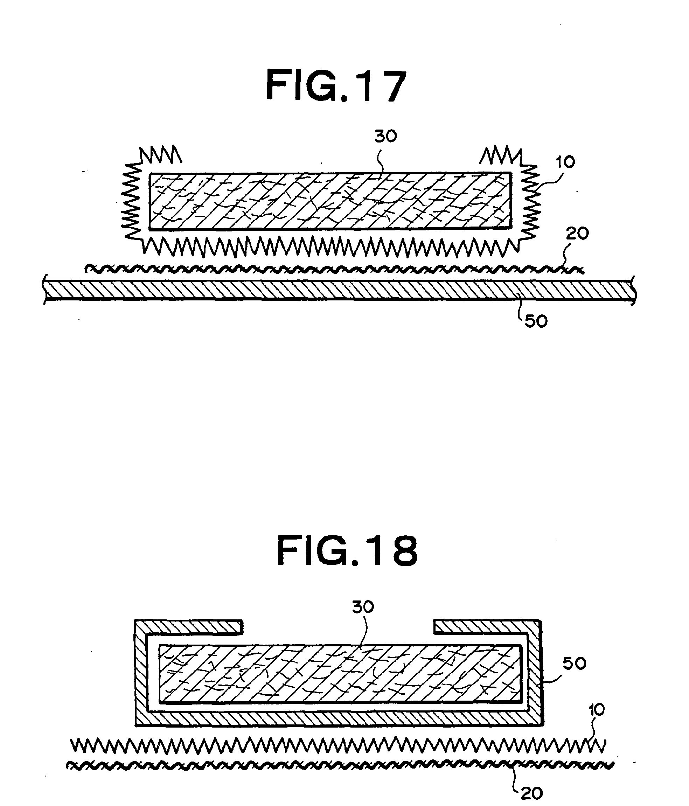

An absorbent water resistant sheet 50 like this is used as a back-up sheet. Embodiments of absorbent products with such back-up sheet added are shown in Figs. 17 and 18.

First of all, in an example shown in Fig. 17, an absorbent core is a mixture of SAP and pulp, in a so-called ultra-thin type structure with approximately 35% of SAP and the balance of fluff pulp. In this case, since the amount of free fluids is relatively large, an absorbent water resistant sheet 50 as shown in Fig. 16 is further added as a back-up sheet under a water resistant barrier sheet comprising a buffer sheet and a water resistant nonwoven fabric. By employing a structure like this, the protectiveness against leakage is considerably improved. Incidentally, in Fig. 17, reference code 10 represents an apertured film, reference code 20 indicates a water resistant nonwoven fabric, and reference code 30 indicates an absorbent core, respectively.

In an example shown in Fig. 18, since an absorbent core 30 is of an absorbent structure mainly consisting of fluff pulp with 10 % of SAP contained, the amount of free fluids is very large. In this case, an absorbent core 30 mainly consisting of fluff pulp is wrapped with an absorbent water resistant sheet 50, and, after free fluids are trapped at a first stage by this absorbent water resistant sheet, the protectiveness against leakage is secured by a highly air permeable and water resistant sheet comprising a buffer sheet and a water resistant nonwoven fabric. The absorbent water resistant sheet used here is excellent in air permeability, so that the high air permeability of an absorbent product, which is a feature of the present invention, is not considerably injured. In Fig. 18, the same or equivalent reference codes are used as and to those used in Fig. 17.

### Air Permeability and Evaluation Methods

In the present invention, an apertured film is a material which is extremely air permeable just like a water resistant nonwoven fabric, and thus its composite of such material, a highly air permeable and water resistant barrier sheet of the present invention, is also extremely air permeable, which is several hundred times as high as that of any conventional air permeable back sheet, which is equivalent to that of any underwear so that the evaluation of its air permeability by WVTR is difficult to perform. Therefore, the air permeability tests were performed by means of air permeability test by ASTM (D-737) and Gurley methods by ISO 5636/5. As a result, almost all examples showed similarly excellent air permeability.

The air permeability tests by ASTM (D-737) were expressed in terms of the amount of air (m³) passing through samples of 1 m² for one minute and Gurley tests were expressed in terms of the time in sec for air of 100 ml to pass through.
· Air permeability tests (m³/min, m²) : 1 m³ or more
· Gurley tests (sec/100ml) : 1 sec or less

From the test results, the behavior of highly air permeable and water resistant barrier sheets according to the present invention exhibiting its water resistance while maintaining their air permeability is understood to be entirely different from the mechanism of air permeability means applied to conventional back sheets.

### Test Methods of Water Resistance

Next, the evaluation of water resistance is explained.

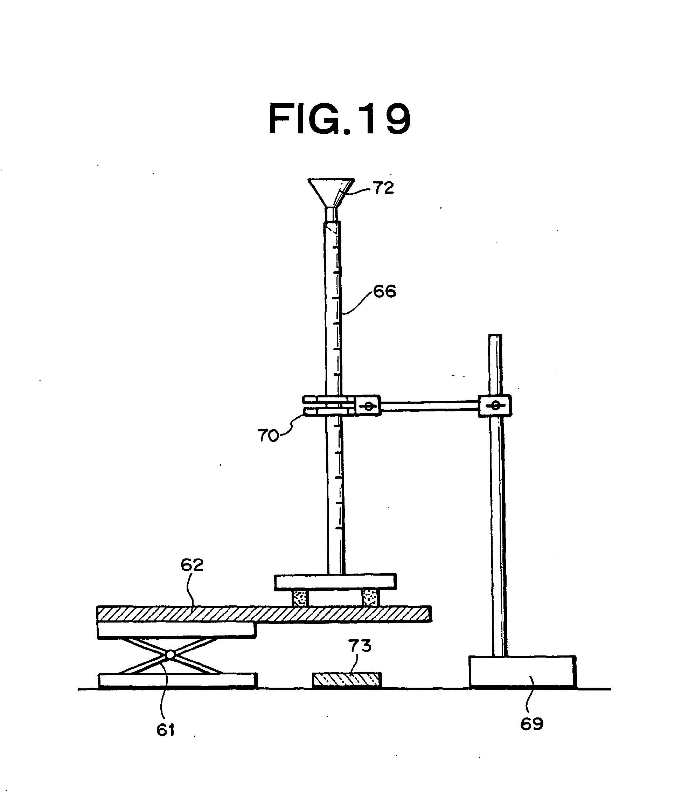

Fig. 19 shows the whole of an evaluation apparatus employed here. Fig. 20 shows an enlargement of the portion where samples for evaluation are mounted of the apparatus.

The sizes of samples are 5 cm×5 cm or larger. The samples were mounted in the following way:
1) Place 2 pieces as folded of filter paper 63 (model: No.2 and size: 15 cm×15 cm, which details are applicable to the other pieces of filter paper.) on a glass plate 62 on a jack 61.
2) Place a sample 64 in the center of the pieces of filter paper 64 with their SAP coated surface facing upwards.
3) Place a piece of tissue 65 (4 cm×4 cm or larger) smaller than the sample on the sample 64.
4) Then, set an acrylic pipe 66 in the following manner.
5) Attach a cushion packing 68 meeting the size of a hole (20 mm φ) on the under surface of an acrylic seat 67 made integrated with the acrylic pipe 66.
6) Hold the acrylic pipe 66 by means of a clump 70 of a stand 69, and adjust so as to have the hole of the bottom of the acrylic pipe 66 meet the center of the sample 64. In this case, hold the acrylic pipe 66 at a right angel to the sample 64.
7) Following the positioning of the acrylic pipe 66, fix the position of the acrylic pipe 66 by tightening the clump 70.
8) Lift the jack 61 so that tightening for prevention of leakage is done by interceding the cushion packing 68 between an acrylic sheet 62 and the acrylic seat 67 of the bottom of the acrylic pipe 66.

Next, in the following procedure the water resistance pressure tests were performed to evaluate the water resistance:

First of all, from the top edge of the acrylic pipe, pour gently a small amount (for example, 2 to 3 ml) of measurement fluid (0.9 **%** NaCl aqueous solution, colored in edible blue No.1) contained in a washing bottle. In this case, make sure that the sample surface has absorbed fluids sufficiently.

Allow the sample to stand for one minute so that SAP particles on the sample surface are swollen. In this case, too, make sure that no measurement fluid has leaked.

Next, attach a funnel 72 on the top edge of the acrylic pipe, and add the measurement fluid. Read the indicated graduation of the acrylic pipe when leakage takes place on the filter paper on the glass plate, which is understood to be the water resistance (P). Read the graduation in terms of 10 mmH₂O.

It is noted that the foregoing evaluation is performed as observation is done appropriately using a mirror 73 disposed under the glass plate 62.

### Examples

(Analysis of absorbing behavior of an absorbent product model made by combining an absorbent core and a highly air permeable and water resistance barrier sheet according to the present invention)

A highly air permeable and water resistant barrier sheet according to the present invention on which an absorbent core was placed as folded to make an absorbent product model. The configuration of this product model is shown in Fig. 21 with reference code 10 representing an apertured film, 20 indicating water resistant nonwoven fabric, 30 indicating an absorbent core and 60 indicating an elastic element, respectively. In addition, on the center top surface of the absorbent core a top sheet 301 and a Liquid distribution unit (LDU) 302 are disposed as necessary. Liquid distribution unit (LDU) in this case uses an apertured film and the smaller the area of use, the higher the effects comparing the top sheet 301 area.

### <Water resistant nonwoven fabric>

As a water resistant nonwoven fabric, SMMS which is a laminate of PP spun bond web and PP melt blown web (of Avgol) was prepared. In the constitution of SMMS, 6 g of S and 4 g of M were used as laminated in an integrated SMMS. The weight was 20 g/m² and the water resistance was 180 mmH₂O.

### <Buffer sheet>

An apertured film sample (B) (prepared by Avgol) as shown in Fig. 5 was used as a buffer sheet. The sheet was so high in air permeability that it showed low resistance to permeability.

### <Absorbent core>

An absorbent core (prepared by Tokushu Paper Ltd.) using MegaThin (registered trademark) wherein a spun lace substrate was coated with SAP was prepared. The absorbent core was manufactured by a process recited in Japanese Patent 3046367, and the constituents of MegaThin were 150 g/m² of SAP and 40 g/m² of a nonwoven fabric substrate with 7 mm and 3 mm (7/3) of SAP coating width and distance resulting in the total weight of 190 g/m². This MegaThin was cut into 100 mm × 270 mm to constitute the absorbent core 30 where the MegaThin was folded as trebled on both sides and a top sheet 301 and a Liquid distribution unit (LDU) 302 were disposed in the center portion. The amount of absorption of the absorbent core 30 was 4500 ml and the retention was 320 ml. The absorbent core had a high air permeability due to the existence of a portion comprising only a nonwoven fabric.

A buffer sheet comprising a PE apertured film 10 was disposed for covering the whole under surface of the absorbent core with its top portion made in contact with the under surface of the absorbent core, and further on the under surface of the buffer sheet a water resistant nonwoven fabric (SMMS) was bonded in an integrated manner.

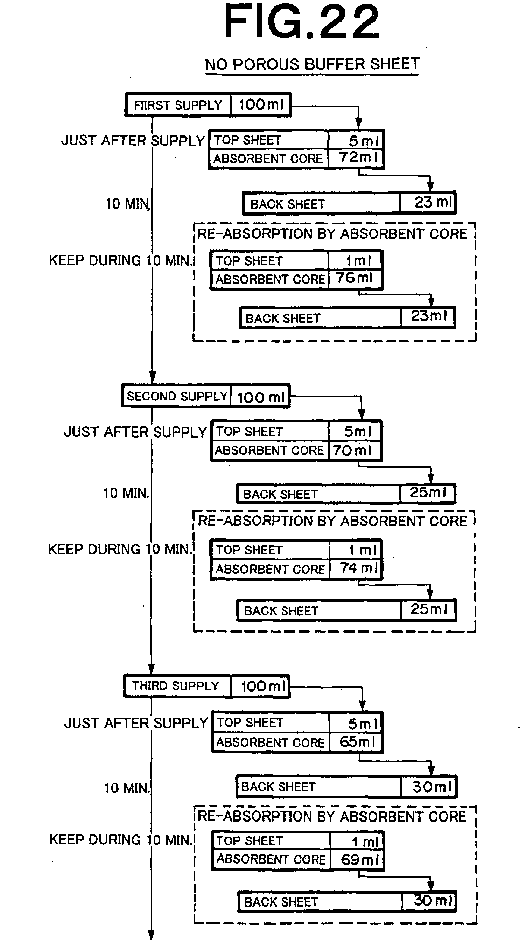

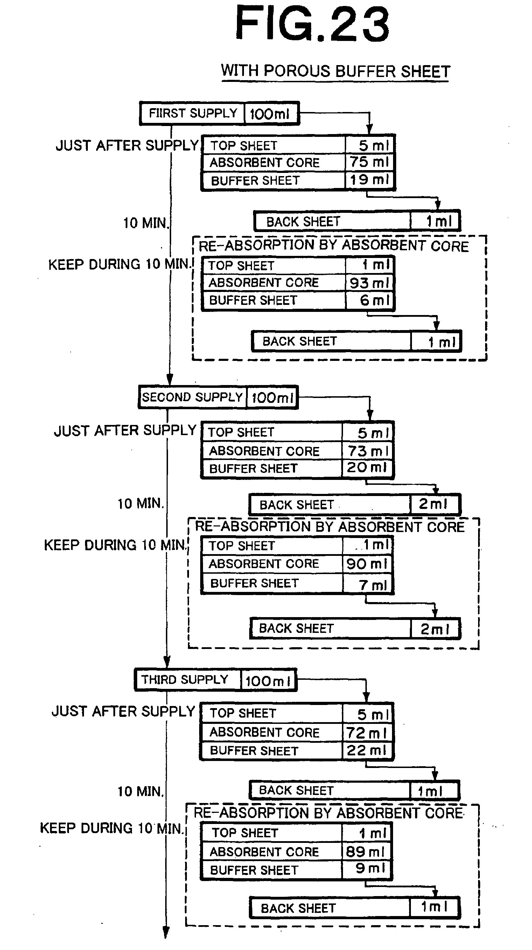

To this product model 300 ml of physiological saline water was added in an amount of 100 cc at 10-minute intervals to analyze the existence condition of fluids in terms of the amount of absorption and the amount transferred to the buffer sheet and to compare the material balances. The results are given in Figs. 22 and 23.

In a system shown in Fig. 22 where no porous buffer sheet was used (get rid of buffer sheet 10 from Fig. 21 product), fluids yet to be absorbed not captured by the absorbent core were flowed out to the water resistant nonwoven fabric side (absorbed by back-up sheet). The change between immediately after time the fluids were absorbed and the time when the absorbent core was allowed to stand for 10 minutes thereafter was only that fluids yet to be absorbed as contained by the top sheet were reabsorbed by the absorbent core. The amounts of fluids not absorbed and discharged were 23 ml at a first time, 25 ml at a second time and 30 ml at a third time. 78 ml in total out of 300 ml was discharged at a breath to the side of the back-up sheet. This flow was neither regulated nor distributed and that a large load was imposed on the buck-as a sheet and possible leakage is likely as pressurized under the body weight. Therefore, a material such as PE film which is highly water resistant and at the same time necessarily less air permeable needed to be employed.

As against this, if one observed in a system shown in Fig. 23 where a porous buffer sheet is combined with a water resistant nonwoven fabric, immediately after a first 100 ml was supplied, 5 ml of fluids yet to be absorbed was trapped by the top sheet, 75 ml was trapped by the absorbent core and 19 ml was trapped by the buffer sheet, and only 1 ml leaking from these elements was further distributed leaked out of the pores to the back-up sheet. Fluids thus leaking were only those existent in drops on the water resistant nonwoven fabric. Of course, no fluids leaked out of the water resistant nonwoven fabric at all. Until when a next supply was made, SAP of the absorbent core swelled to restore an absorbing capacity and reabsorbed by absorbing fluids yet to be absorbed out of space A and space B as mentioned above which existed around the buffer sheet, thereby the spaces around the buffer sheet became empty to be ready to receive a next supply of fluids. The amounts of fluids not absorbed and discharged were 1ml at a first time, 2ml at a second time and 1ml at a third time.

As a second supply of fluids started, a cycle similar to that at the first time was repeated. At the time that a third absorption of fluids was finished, 5 ml out of the total of 300 ml was re-divided and became existent in sheets as a load to the water resistant sheet.

As a result, the following differences were observed depending upon whether a buffer sheet is provided or not:
(1) The load to the buffer sheet side was considerably reduced from 78 ml to 5 ml.
(2) As against the fact that without a buffer sheet used the load of 78 ml was concentrated on a part of the back-up sheet, the use of a buffer sheet according to the present invention re-distributed the load as applied to the back-up sheet of 5 ml further into one of several tenths.
(3) When no buffer sheet was employed, the body weight of a wearer was applied to the back-up sheet as a direct water pressure, but as the pressure was dissipated and alleviated by the use of the porous buffer sheet thereby the pressure was made indirectly uniform, so that the pressure as applied to the back-up sheet was made very little.

On observation of the leakage of an absorbent product model provided with the above-described buffer sheet and water resistant nonwoven fabric being made to absorbed 300 ml while was worn actually at a sitting position and then allowed to stand for 5 minutes with 50 kg body weight applied, no leakage took place at all.

A highly air permeable and water resistant barrier sheet according to the present invention has a high air permeability and at the same time a high level of water resistance. Absorbent products having this barrier sheet applied therein as a back sheet such as baby diapers, adult diapers, feminine hygiene products and articles for pets provide an excellent and comfortable feeling of use with much less skin rash and stuffiness.

### Industrial Applicability

A highly air permeable and water resistant barrier sheet has not only a high air permeability but also a good water resistance, and as such is applicable for use as water barrier in baby diapers, adult diapers and any other absorbent products.

## Claims

1. A highly air permeable and water resistant barrier sheet comprising a composite obtained by bonding, in an integrated form,
a porous buffer sheet which has pores having a diameter of 1 mm or less and has a number of 10 pores/cm² or more and
a water resistant nonwoven fabric having a water resistance value of 100 mmH₂O or more.

2. A highly air permeable and water resistant barrier sheet according to claim 1 wherein said porous buffer sheet comprises a highly air permeable foam sheet having continuous air bubbles and having a number of 20 cells/25 mm or more and a thickness of 3 mm or less.

3. A highly air permeable and water resistant barrier sheet according to claim 1 wherein said porous buffer sheet is an apertured film with many funnel-shaped hollow juts,
the diameter of the top portion of said juts being different from that of the bottom portion of said juts,
the diameter of the top portion of said juts being 1 mm or less when the shape of the top portion of said juts is approximated to a perfect circle,
said porous buffer sheet having a number of 10 pores/cm² or more.

4. A highly air permeable and water resistant barrier sheet according to either of claims 1 to 3 wherein said water resistant nonwoven fabric is a multi-layered member of a spun bond web and a melt blown web which are formed by using, as a raw material base, PE, PP, PET or polyurethane and their derivatives alone or in any arbitrary combination.

5. A highly air permeable and water resistant barrier sheet according to claim 4 wherein said multi-layered member of spun bond (S) and melt blown (M) has a layer configuration selected from the group consisting of SMS, SMMS, SMMMS and SMSMS.

6. An absorbent product having an absorbent core including at least one of SAP component and fluff pulp component in the form of a powder, particulate or fiber as an absorbent component,
a highly air permeable and water resistant barrier sheet being provided and disposed in contact with the layer of said absorbent component,
said highly air permeable and water resistant barrier sheet comprising a composite obtained by bonding, in an integrated form, a porous buffer sheet which has pores having a diameter of 1 mm or less and has a number of 10 pores/cm² or more and
a water resistant nonwoven fabric having a water resistance value of 100 mmH₂O or more.

7. An absorbent product according to claim 6 wherein said buffer sheet is an apertured film with many funnel-shaped hollow juts, the diameter of the top portion of said juts being different from that of the bottom portion of said juts,
the diameter of the top portion of said juts being 1 mm or less when the shape of the top portion of said juts is approximated to a perfect circle,
said porous buffer sheet having a number of 10 pores/cm² or more, the top portion of said juts being disposed in contact with said absorbent core.

8. An absorbent product according to either of claims 6 and 7 wherein said buffer sheet is disposed to cover the whole undersurface and both sides of said absorbent core and a part of the surface of said absorbent core,
the undersurface of said absorbent core being bonded, in an integrated form, to said water resistant nonwoven fabric.

9. An absorbent product according to either of claims 6 and 7 wherein said buffer sheet covers the whole undersurface and both sides of said absorbent core and is bonded, in an integrated form, to said water resistant nonwoven fabric in the undersurface of said absorbent core.

10. An absorbent product according to either of claims 6 and 7 wherein said buffer sheet is disposed, developed on the undersurface of said absorbent core, and bonded, in an integrated form, to said water resistant nonwoven fabric on the whole surface of said absorbent core so that said buffer sheet and said water resistant nonwoven fabric may function as a back-up sheet.

11. An absorbent product according to either one of claims 6 to 10 wherein a non air permeable film of high water resistance or an air permeable film of high water resistance is added to reinforce the portion of the whole absorbing surface of said absorbent core where discharged body fluids are intensively supplied in excess of the water retaining capability of said absorbent core or pressure is concentrated locally.

12. An absorbent product according to claim 11 wherein said highly water resistant film and said buffer sheet are combined with each other only in their respective edges folded on each other and are bonded in an integrated form.

13. An absorbent product according to either one of claims 6 to 11 wherein an absorbent water resistant sheet is further added to said barrier sheet as a back-up sheet in order to improve the water resistance stability thereof.

14. An absorbent product according to claim 13 wherein said absorbent water resistant sheet has a three-layered structure as composed of said water resistant nonwoven fabric, a hydrophilic sheet having a water dispersibility and a SAP layer as interposed between said water resistant sheet and said hydrophilic sheet.

15. An absorbent product according to claim 14 wherein said absorbent water resistant sheet has a thickness of 0.5 mm or less, said water resistant nonwoven fabric is in the form of SMS or SMMS of 30 g/m² to 10 g/m², said hydrophilic sheet is a tissue paper mainly comprising wood pulp of 30 g/m² to 10 g/m² and said SAP layer includes SAP particles of 80 g/m² to 210 g/m² whose particle size is 500 µm or less.

16. An absorbent product according to either one of claims 6 to 15 wherein 50% or more of the whole weight of said absorbent core is composed of said SAP particles.

17. An absorbent product according to either one of claims 6 to 15 wherein said absorbent core is a sheet-like composite comprising a nonwoven fabric and SAP particles as carried by said nonwoven fabric.

18. An absorbent product according to either one of claims 6 to 15 wherein said absorbent core comprises a mixture of fluff pulp and SAP particles and is a sheet-like absorbent body formed by an air laid method.

## Amended claims

### Amended claims under Art. 19.1 PCT

**1.** (Amended) A highly air permeable and water resistant barrier sheet comprising a composite obtained by bonding, in an integrated form,
a porous buffer sheet which has pores having a diameter of 1 mm or less and has a number of 10 pores/cm² or more and
a water resistant nonwoven fabric having a water resistance value of 100 mmH₂O or more, said water resistant nonwoven fabric comprising a spun bond web and a melt blown web.

**2.** A highly air permeable and water resistant barrier sheet according to claim 1 wherein said porous buffer sheet comprises a highly air permeable foam sheet having continuous air bubbles and having a number of 20 cells/25 mm or more and a thickness of 3 mm or less.

**3.** (Amended) A highly air permeable and water resistant barrier sheet according to claim 1 wherein said porous buffer sheet is an apertured film with many funnel-shaped hollow juts,
the diameter of the top portion of said juts being different from that of the bottom portion of said juts,
the diameter of the top portion of said juts being 1 mm or less when the shape of the top portion of said juts is approximated to a perfect circle,
said porous buffer sheet having a number of 10 pores/cm² or more, said water resistant nonwoven fabric being bonded to said bottom portion of said hollow juts of said porous buffer sheet.

**4.** (Amended) A highly air permeable and water resistant barrier sheet according to either of claims 1 to 3 wherein said water resistant nonwoven fabric is formed by using, as a raw material base, PE, PP, PET or polyurethane and their derivatives alone or in any arbitrary combination.

**5.** A highly air permeable and water resistant barrier sheet according to claim 4 wherein said multi-layered member of spun bond (S) and melt blown (M) has a layer configuration selected from the group consisting of SMS, SMMS, SMMMS and SMSMS.

**6.** (Amended) An absorbent product having an absorbent core including at least one of SAP component and fluff pulp component in the form of a powder, particulate or fiber as an absorbent component,
a highly air permeable and water resistant barrier sheet being provided and disposed in contact with the layer of said absorbent component,
said highly air permeable and water resistant barrier sheet comprising a composite obtained by bonding, in an integrated form,
a porous buffer sheet which has pores having a diameter of 1 mm or less and has a number of 10 pores/cm² or more and
a water resistant nonwoven fabric having a water resistance value of 100 mmH₂O or more,
said water resistant nonwoven fabric comprising a spun bond web and a melt blown web.

**7.** An absorbent product according to claim 6 wherein said buffer sheet is an apertured film with many funnel-shaped hollow juts,
the diameter of the top portion of said juts being different from that of the bottom portion of said juts,
the diameter of the top portion of said juts being 1 mm or less when the shape of the top portion of said juts is approximated to a perfect circle,
said porous buffer sheet having a number of 10 pores/cm² or more, the top portion of said juts being disposed in contact with said absorbent core.

**8.** An absorbent product according to either of claims 6 and 7 wherein said buffer sheet is disposed to cover the whole undersurface and both sides of said absorbent core and a part of the surface of said absorbent core,
the undersurface of said absorbent core being bonded, in an integrated form, to said water resistant nonwoven fabric.

**9.** An absorbent product according to either of claims 6 and 7 wherein said buffer sheet covers the whole undersurface and both sides of said absorbent core and is bonded, in an integrated form, to said water resistant nonwoven fabric in the undersurface of said absorbent core.

**10.** (Amended) An absorbent product according to either of claims 6 and 7 wherein said buffer sheet is disposed, developed on the undersurface of said absorbent core, and bonded, in an integrated form, to said water resistant nonwoven fabric on the whole surface of said absorbent core so that said buffer sheet and said water resistant nonwoven fabric may function as a water resistant barrier sheet.

**11.** An absorbent product according to either one of claims 6 to 10 wherein a non air permeable film of high water resistance or an air permeable film of high water resistance is added to reinforce the portion of the whole absorbing surface of said absorbent core where discharged body fluids are intensively supplied in excess of the water retaining capability of said absorbent core or pressure is concentrated locally.

**12.** (Amended) An absorbent product according to claim 11 wherein said highly water resistant film and said buffer sheet are combined with each other only in their respective edges folded on each other and are bonded in an integrated form.

**13.** An absorbent product according to either one of claims 6 to 11 wherein an absorbent water resistant sheet is further added to said barrier sheet as a back-up sheet in order to improve the water resistance stability thereof.

**14.** An absorbent product according to claim 13 wherein said absorbent water resistant sheet has a three-layered structure as composed of said water resistant nonwoven fabric, a hydrophilic sheet having a water dispersibility and a SAP layer as interposed between said water resistant sheet and said hydrophilic sheet.

**15.** An absorbent product according to claim 14 wherein said absorbent water resistant sheet has a thickness of 0.5 mm or less, said water resistant nonwoven fabric is in the form of SMS or SMMS of 30 g/m² to 10 g/m², said hydrophilic sheet is a tissue paper mainly comprising wood pulp of 30 g/m² to 10 g/m² and said SAP layer includes SAP particles of 80 g/m² to 210 g/m² whose particle size is 500 µm or less.

**16.** An absorbent product according to either one of claims 6 to 15 wherein 50% or more of the whole weight of said absorbent core is composed of said SAP particles.

**17.** An absorbent product according to either one of claims 6 to 15 wherein said absorbent core is a sheet-like composite comprising a nonwoven fabric and SAP particles as carried by said nonwoven fabric.

**18.** An absorbent product according to either one of claims 6 to 15 wherein said absorbent core comprises a mixture of fluff pulp and SAP particles and is a sheet-like absorbent body formed by an air laid method.
